# EUROPEAN PATENT APPLICATION

(11) **EP 4 470 564 A1**
(43) Date of publication of application: **04.12.2024**
(21) Application number: 23743010.3
(22) Date of filing: 15.02.2023
(51) Int. Cl.: A61K 45/06, A61K 38/16, A61K 38/21, A61K 31/675, A61K 31/513, A61K 31/7072, A61K 31/522, A61P 1/16, A61P 31/20, A61P 31/14

(54) **DRUG AND METHOD FOR NUCLEOSIDE (ACID) DRUG THERAPY OF LIMITING CHRONIC HEPATITIS B**

(30) Priority: 24.01.2022 CN 202210079582
(71) Applicant: Shanghai Hep Pharmaceutical Co., Ltd., Pudong New Area, Shanghai 201203 (CN)
(72) Inventor: LIU, Hongli, Shanghai 201203 (CN)
(74) Representative: V.O.
(86) International application number: PCT/CN2023/076223
(87) International publication number: WO 2023/138696

(57) **Abstract**

The present invention relates to a drug and method for nucleoside (acid) drug therapy of limiting chronic hepatitis B, in particular to the use of an HBV virus entry inhibitor and an optional immunomodulator in the preparation of a drug or kit for nucleoside (acid) drug therapy of limiting or terminating chronic hepatitis B. By using the method provided by the invention, after administering the nucleoside (acid) drug, the HBV virus entry inhibitor and the optional immunomodulator for combined therapy for a certain course of treatment, the drug can be completely stopped, and the patient does not need to continue to accept NAs treatment.

## Description

### TECHNICAL FIELD

The present description relates to a medicine and method for nucleoside (acid) therapy of limiting chronic hepatitis B.

### BACKGROUND

Currently only nucleoside (acid) medicines (NAs) and interferon are approved for the treatment of chronic hepatitis B. Nucleoside (acid) medicines inhibitHBV replication by inhibiting HBV DNA polymerase. Nucleoside (acid) medicines currently approved by the FDA for the treatment of hepatitis B comprise lamivudine, adefovir dipivoxil, entecavir, tenofovir disoproxil (TDF), telbivudine, and tenofovir alafenamide hemifumarate (TAF). After the treatment with nucleoside (acid) medicines, the level of viral DNA in patients is well suppressed. For example, tenofovir, which was the new generation of NAs, has improved safety, and presents an inhibition rate of about 70% on viral DNA. Although nucleotide medicines can effectively inhibit virus replication, long-term medication is required, and relapse and rebound often occur after medicine withdrawal. Therefore, NAs treatment for hepatitis B is an infinite therapy.

TAF is an antiviral medicine developed by Gilead, which was approved by the FDA in 2016 for the treatment of chronic hepatitis B. TAF is a novel nucleotide reverse transcriptase inhibitor. After entering the liver cells, TAF can be hydrolyzed into tenofovir, and then be phosphorylated to block virus replication. As an upgraded version of tenofovir disoproxil TDF, TAF retains TDF's advantages of "highly effective antiviral and low medicine resistance", and overcomes TDF's shortcomings of partial nephrotoxicity and bone density damage. Compared with TDF, the dosage of TAF is lower for the same curative effect, and the risk of osteoporosis and nephrotoxicity is smaller. Although TAF has many advantages, it still has the problem of needing long-term administration. There is currently no uniform standard for discontinuation of nucleotide analogues. According to the recommendations in the "Guidelines for the Prevention and Treatment of Chronic Hepatitis B (2019)": for HBeAg(+) chronic hepatitis B patients, if 1 year of treatment leads to HBV DNA being lower than the lower limit of detection, ALT returning to normal and HBeAg seroconversion, continued treatment for at least 3 years (recheck every 6 months) is required; then If the above indicators remain unchanged, treatment withdrawal can be considered. For HBeAg(-) patients, it is recommended to stop medicine and be followed up after HBsAg disappears and HBV DNA cannot be detected. It is almost impossible to realize the disappearance of HBsAg with nucleoside (acid) medicines alone. A large number of clinical data also show that the relapse rate after discontinuation of nucleoside medicines is high. For HBeAg-positive and negative patients, factors such as patient age, HBV DNA level, and consolidation time all affect the recurrence rate after medicine discontinuation. How to achieve safe discontinuation of NAs medicines is an urgent need for the majority of patients treated with NAs, and there is an urgent need for a method of finitelization of infinite NAs therapy.

Interferon is a cytokine with a broad-spectrum antiviral effect. It was originally used in the treatment of HIV and other viruses. In 1992, it was approved by the FDA for the treatment of hepatitis B. Interferon acts against viruses mainly through immune regulation and induction of antiviral proteins in liver cells. The meta-analysis showed that after 4 to 6 months of treatment of HBeAg-positive patients with common interferon a (common IFNα), the HBV DNA negative rate (hybrid method) in the treatment group and the non-treated group was 37% and 17%, respectively; and the HBeAg negative rate in the treatment group and the non-treated group was 33% and 12%, respectively; and the HBsAg negative rate in the treatment group and the non-treated group was 7.8% and 1.8%, respectively. The curative effect was positively correlated with the baseline serum ALT level and the degree of liver histological lesions. Four randomized controlled trials on HBeAg-negative patients showed that the response rate at the end of treatment was 38% to 90%, but the persistent response rate was only 10% to 47% (average 24%). Better curative effect requires at least 1 year of ordinary IFNα treatment. International multi-center randomized controlled clinical trials showed that, after pegylated interferon α-2a (PegIFNα-2a) (40KD) treatment for 48 weeks and followed up for 24 weeks after medicine withdrawal, the HBeAg seroconversion rate of HBeAg-positive chronic hepatitis B patient (87% Asians) was 32%, and 43% of the HBeAg-negative patients (60% Asians) shows HBV DNA <2×10⁴ copies/ml (42% after followed up for 24 weeks).

In recent years, with the long-term follow-up of patients taking interferon, it has been found that although there are many adverse events, the long-term benefits of interferon therapy have become more and more clear. After a course of treatment with interferon alone, the negative conversion rate of viral surface antigen continued to increase with the extension of follow-up time (Hannah S.J.Choi, et al. Clinical Gastroenterology and Hepatology, 2020). The adverse reaction of interferon therapy is serious under the current dosage, and the patient's compliance with treatment is poor. These adverse reactions comprise influenza-like syndrome, manifested as fever, chills, headache, muscle aches, and fatigue; transient peripheral blood cell reduction, mainly manifested as a decrease in peripheral blood leukocytes (neutrophils) and thrombocyte; mental abnormalities, manifested as depression, delusion, severe anxiety and other mental symptoms; autoimmune diseases, some patients might have autoantibodies, only a small number of patients had thyroid disease (hypothyroidism or hyperthyroidism), diabetes, thrombocytopenia, psoriasis, leukoplakia, Rheumatoid arthritis and systemic lupus erythematosus-like syndrome; other rare adverse reactions, comprising renal damage (interstitial nephritis, nephrotic syndrome and acute renal failure, etc.), cardiovascular complications (arrhythmia, ischemic heart disease and myocardiopathy, etc.), retinopathy, hearing loss, and interstitial pneumonia, etc. The current standard course of interferon therapy is 48 weeks.

In order to achieve a limited course of treatment and get rid of the limitation of long-term use of nucleoside medicines, industry and academia have tried to use interferon and nucleoside medicines in a combined treatment or a sequential treatment, but no increase in efficacy has been found. Some academic studies have shown that the combination of interferon and tenofovir seems to be better than monotherapy, but the long-term treatment of this combination is not clear, and it has not been promoted and included in the guidelines in clinical practice (M Vigano, et al. Alimentary Pharmacology and Therapeutics, 44(7), 653-661).

Viral entry inhibitors comprise polypeptides derived from the Pre-S 1 region of HBV and anti-Pre-S 1 antibodies or anti-surface antigen antibodies. Polypeptides derived from the HBV Pre-S 1 region include, but are not limited to, hepalatide and bulevirtide. The amino acid sequence of hepalatide is derived from the amino acids 13-59 of the Pre-S 1 region of HBV genotype C, and its N-terminus is modified with myristic acid. Studies have shown that sodium taurocholate co-transporting polypeptide (NTCP) is a receptor for HBV infection. HBV specifically binds to NTCP through the Pre-S 1 region of the large protein on the surface of the viral envelope, and mediates HBV infection of liver cells. Hepalatide and bulevirtide can specifically bind to NTCP and block HBV infection of liver cells *in vitro* and *in vivo.*

### SUMMARY

The first aspect of the present description provides the use of an HBV virus entry inhibitor and an optional immunomodulator in the preparation of a medicament or kit for limiting or terminating nucleoside (acid) treatment of chronic hepatitis B.

In one or more embodiments, the HBV virus entry inhibitor is selected from the group consisting of: HBV entry inhibitory polypeptides derived from the HBV Pre-S1 region, or HBV Pre-S 1 derived peptides, anti-Pre-S 1 antibodies, anti-surface antigen antibodies or other agents that can inhibit HBV from entering or infecting liver cells.

In one or more embodiments, the HBV entry inhibitory polypeptide derived from HBV Pre-S1 region is derived from the pre-S1 region of the surface antigen of any one of HBV genotypes A, B, C, D, E, F, G, H and I.

In one or more embodiments, the HBV entry inhibitory polypeptide derived from the HBV Pre-S1 region is 10-118 amino acid residues in length.

In one or more embodiments, the immunomodulator comprises an agent selected from the group consisting of: interferon, toll-like receptor agonist, CPG, CPG ODN, PD-1 inhibitor, PD-L1 inhibitor, interleukin and cytokine.

In one or more embodiments, the interferon is selected from IFN-α, IFN-β and IFN-γ, preferably IFNα-2a and/or IFNα-2b, more preferably PEG IFNα-2a and/or PEG IFNα-2b.

In one or more embodiments, the HBV entry inhibitory polypeptide derived from the HBV Pre-S 1 region is derived from the pre-S1 region of the HBV genotype C surface antigen; preferably, the HBV entry inhibitory polypeptide is the fragment or variant thereof of N-terminus of the pre-S1 region of the HBV genotype C surface antigen , the fragment at least comprises the amino acid residues 13-44 of the N-terminus; preferably, the HBV entry inhibitory polypeptide comprises the amino acid residues 2-119, 2-69, 2-59, 13-119, 13-88, 13-72, 13-67, 13-59, 13-52 or 13-47 of the pre-S1 region of the HBV genotype C surface antigen, or a variant thereof.

In one or more embodiments, the HBV entry inhibitory polypeptide derived from the HBV Pre-S 1 region is a fragment of the N-terminus of the pre-S1 region of a surface antigen of HBV genotype A, B, F, H or I, or a variant thereof, the fragment comprises at least amino acid residues 13-44 of the N-terminus, preferably comprises at least amino acid residues 13-69 of the N-terminus.

In one or more embodiments, the HBV entry inhibitory polypeptide derived from the HBV Pre-S 1 region is a fragment of the N-terminus of the pre-S1 region of the HBV genotype D surface antigen, or a variant thereof, and the fragment comprises at least the amino acid residues 2-33 of the N-terminus, preferably comprises at least the amino acid residues 2-48 of the N-terminus.

In one or more embodiments, the HBV entry inhibitory polypeptide derived from the HBV Pre-S1 region is a fragment of the N-terminus of the pre-S1 region of the HBV genotype E, G surface antigen, or a variant thereof, and the fragment comprises at least the amino acid residues 12-43 of the N-terminus, preferably comprises at least the amino acid residues 12-68 of the N-terminus.

In one or more embodiments, compared with the fragment, the variant has deletions, substitutions or insertions of 1-30 amino acid(s), and the variant retains the biological activity of inhibiting HBV entry or binding to NTCP.

In one or more embodiments, the variants comprise natural flanking amino acid sequences from any one of the HBV subtypes inserted at the ends of N- and/or C-terminus of the fragments.

In one or more embodiments, the natural flanking amino acid sequence is selected from: the amino acids 2-12 of the N-terminus of the pre-S1 region of the HBV genotypes A, B, F, H, I surface antigen and the amino acids derived from the amino acid 2-11 of the N-terminus of the pre-S1 region of HBV genotypes E, G surface antigen.

In one or more embodiments, the variant is a variant that obtained by introducing one or combination of any two or more of the following amino acid substitutions into a derivative peptide derived from the amino acids 13-59 of the pre-S1 region of HBV genotype C: N15D, F25L, G35K, N39E, F45L, N46K, N48H or N48Y or N48K, D50A, H51Q or H51N, E54K or E54D, A55S, N56K or N56D, and Q57K, optionally, the N- and/or C-terminus of the variant inserted into the natural flanking amino acid sequence of any of the HBV subtypes.

In one or more embodiments, the amino acid sequence of the fragment is as shown in any one of SEQ ID NO: 2-32, or has at least 30%, 40%, 50%, 60%, 70%, 80%, 85%, 90%, 95%, 96%, 97%, 98% or 99% identity with the amino acid sequence as shown in any one of SEQ ID NO: 2-32.

In one or more embodiments, the HBV entry inhibitory polypeptide derived from the HBV Pre-S 1 region has N-terminus modification and/or C-terminus modification.

In one or more embodiments, the N-terminus modification is a hydrophobic group modification.

In one or more embodiments, the hydrophobic group modification is selected from a modification by the group consisting of: myristic acid, palmitic acid, stearic acid, cholesterol, oleic acid, linoleic acid, polyethylene glycol, and arachidonic acid.

In one or more embodiments, the C modification is amidation or isoprenylation modification.

In one or more embodiments, the HBV entry inhibitory polypeptide derived from the HBV Pre-S 1 region is shown by SEQ ID NO: 33-44.

In one or more embodiments, the use is that HBV virus entry inhibitors, nucleoside (acid) medicines for the treatment of chronic hepatitis B and optional immunomodulators in the preparation of a medicament or a kit for limiting or terminating chronic hepatitis B nucleoside (acid) therapy.

In one or more embodiments, the nucleoside (acid) medicine is selected from the group consisting of: lamivudine, adefovir dipivoxil, entecavir, tenofovir dipivoxil, telbivudine and tenofovir alafenamide hemifumarate, preferably is tenofovir alafenamide hemifumarate.

In one or more embodiments, the kit comprises 1 dose or 2 doses of the above medicament, so as to meet the daily dose of 2.1-10.5 mg, preferably 4.2-8.4 mg, more preferably 4.2 mg of the HBV virus entry inhibitors.

In one or more embodiments, the kit also optionally comprises 1 dose or 2 doses of the above mentioned interferon, and its content meets the dose of 1-360 micrograms per week, preferably 30-180 micrograms per week, more preferably 60-135 micrograms per week, more preferably 90 micrograms per week.

In one or more embodiments, the kit is used for combined administration with the nucleoside (acid) medicine for 1-96 weeks, preferably 12-60 weeks, more preferably 24-48 weeks.

The second aspect of the present description provides a method for limiting or terminating nucleoside (acid) treatment of chronic hepatitis B patients, the method comprising combined treatment of administering chronic hepatitis B patients a HBV virus entry inhibitor and optional an immunomodulator together with a nucleoside (acid) medicine , and after a certain course of combined treatment, nucleoside (acid) therapy, HBV virus entry inhibitor therapy and the immunomodulator therapy are terminated.

In one or more embodiments, the nucleoside (acid) medicine is as described in any embodiment herein; the HBV virus entry inhibitor is as described in any embodiment herein, and the immunomodulator is as described in any embodiment herein.

In one or more embodiments, the duration of the combined treatment is 1-96 weeks, preferably 12-60 weeks, more preferably 24-48 weeks.

In one or more embodiments, before the combined treatment, the patient has been administered by continuous NAs treatment, preferably has been administered by NAs treatment for more than 0 months, more than 3 months, more than 12 months, more than 36 months.

In one or more embodiments, the daily dose of the HBV virus entry inhibitor is 2.1-10.5 mg, preferably 4.2-8.4 mg, more preferably 4.2 mg.

In one or more embodiments, the immunomodulator is interferon, with a weekly dose of 1-360 micrograms, preferably 30-180 micrograms, more preferably 60-135 micrograms, more preferably 90 micrograms.

In one or more embodiments, the nucleoside (acid) medicine is tenofovir alafenamide hemifumarate, and the HBV virus entry inhibitor is hepalatide or bulevirtide.

In one or more embodiments, the treatment period of the hepalatide or bulevirtide is from 1 week to 96 weeks.

In one or more embodiments, the therapeutic dose of hepalatide or bulevirtide is 2.1 mg to 8.4 mg per day.

In one or more embodiments, the immunomodulator is PEG interferon, with a dosage of preferably 10-170 ug per week.

In one or more embodiments, in the method, the nucleoside (acid) medicine is tenofovir alafenamide hemifumarate; combined with hepalatide or bulevirtide with the treatment period of 48 weeks and the dose of 4.2 mg per day; at the same time, PEG interferon is administered with the dose of 90 ug per week and the treatment period of 48 weeks.

### Description of drawings

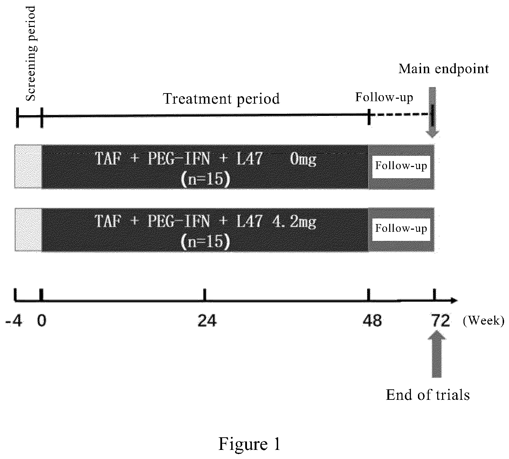

Figure 1: Flowchart of multicenter phase II clinical trial of treatment of chronic hepatitis B by TAF combined with PEG interferon and hepalatide in a limited course (randomized, double-blind, placebo-controlled).

### DETAILED DESCRIPTION

It should be understood that within the scope of the present invention, the above-mentioned technical features of the present invention and the technical features specifically described in the following (such as the examles) can be combined with each other to form a preferred technical solution.

At present, NAs are the main medicine for the treatment of HBV chronic infection, but the treatment is not a limited course of treatment, and HBV rebound and recurrence occur after medicine withdrawal. The invention provides a method capable of limiting NAs treatment, so that chronic hepatitis B (CHB) patients receiving NAs treatment can safely withdraw the medicine, avoid HBV recurrence, and make NAs a limited course of treatment medicine.

The limited NAs treatment method provided by the present invention is: administering CHB patients with combined treatment of an HBV entry inhibitor and continuous NAs treatment, and after a certain course of combined treatment, terminating both NAs and HBV entry inhibitor treatment.

The present invention finds that, with the method of the present invention, the HBV of CHB patients does not rebound after termination of treatment. It should be understood that the term "limitation" or "limited" mentioned herein means that the regimen of the present invention can limit the course of NAs treatment, meet the criteria for NAs treatment withdrawal, and there will be no rebound or recurrence of HBV after withdrawal.

In the present invention, the continuous NAs treatment administered to CHB patients include but are not limited to lamivudine, adefovir dipivoxil, entecavir, TDF, telbivudine, TAF and other NAs drugs.

In a preferred embodiment, the NAs treatment administered to CHB patients are entecavir, TDF, telbivudine or TAF.

In some embodiments, the continuous NAs treatment administered to the CHB patient is the NAs treatment that has been administered for more than 0 months, preferably more than 3 months, more preferably more than 12 months, and further preferably more than 36 months.

The terms "HBV entry inhibitory polypeptide derived from the HBV Pre-S 1 region" or "HBV Pre-S 1 derived peptide" as used herein refers to a polypeptide originating from or derived from the Pre-S1 region of HBV surface antigen and capable of inhibiting HBV virus entry into liver cells, including but not limited to natural, recombinant, synthetic or purified polypeptides, including but not limited to full-length Pre-S1 region of natural HBV surface antigen or their fragments and variants thereof, full-length Pre-S1 region of non-natural HBV surface antigen or their fragments and variants thereof, non-full-length Pre-S1 regions of natural HBV surface antigen or their fragments and variants thereof, non-full-length Pre-S 1 regions of non-natural HBV surface antigen or their fragments and variants thereof, and other forms of variants.

In some embodiments, the HBV entry inhibitory polypeptide derived from the HBV Pre-S1 region may contain the entire surface antigen pre-S 1 region from the second amino acid G (glycine) of the surface antigen pre-S1 region of HBV of any genotype. In certain embodiments, the HBV entry inhibitory polypeptide derived from HBV Pre-S1 region described herein may be derived from the pre-S1 region of the surface antigen of any one of HBV genotypes A, B, C, D, E, F, G, H and I.

Exemplary genome sequences for these HBV genotypes can be found, eg, at GenBank Accession Nos. KC875260, AY220704, AF461363, AY796030, AB205129, DQ823095, HE981176 and AB179747.

In certain embodiments, the HBV Pre-S 1 derived peptides used include but are not limited to HBV virus entry inhibitory polypeptides derived from the amino acid sequence of the surface antigen Pre-S1 region of HBV genotype C, including but not limited to the amino acid sequence SEQ ID NO: 1 of the exemplary HBV surface antigen Pre-S1 region and its variants.

The HBV entry-inhibiting polypeptide derived from the HBV Pre-S1 region at least retains the biological activity of inhibiting HBV virus entry or binding to NTCP.

In some embodiments, the HBV entry inhibitory polypeptide derived from the HBV Pre-S1 region described herein is 10-118 amino acid residues in length.

For example, the polypeptide may be, but not limited to, 15-100, 15-80, 20-100, 20-80, 20-60, 25-60, 30-60, 35-60, or 40-60 (including all integers in these ranges) amino acids in length.

In some embodiments, the HBV entry-inhibiting polypeptides derived from the HBV Pre-S1 region described herein may be at least 20 amino acids in length, eg, at least 25, 30, 35 or 40 amino acids in length. In some embodiments, the HBV entry-inhibiting polypeptides derived from the HBV Pre-S1 region described herein may be but are not limited to 20, 25, 30, 35, 40, 47, 55 or 60 amino acids in length.

In some embodiments, the HBV entry inhibitory polypeptide derived from the HBV Pre-S1 region described herein may be 47 amino acids in length.

The variants of different lengths derived from the entry inhibitory polypeptides of HBV Pre-S1 region HBV described herein retain one or more biological activities related to the corresponding polypeptides, at least including biological activities that can inhibit HBV virus entry or bind to NTCP.

In certain embodiments, the HBV entry-inhibiting polypeptide derived from the HBV Pre-S1 region described herein may be derived from the pre-S1 region of the surface antigen of HBV genotype C, including but not limited to amino acids 2-119, 2-69, 2-59, 13-119, 13-88, 13-72, 13-67, 13-59, 13-52, 13-47, 13-42, 13-37, 13-32 of the pre-S1 region and variants thereof, exemplary sequences include but not limited to SEQ ID NO: 2-14 and variants thereof.

In some embodiments, the HBV entry-inhibiting polypeptide derived from the HBV Pre-S1 region comprises the amino acid sequence of at least amino acid residues 13-44 at the N-terminal of the pre-S1 region of surface antigen of HBV genotype C described herein and variants thereof.

In some embodiments, the HBV entry inhibitory polypeptide derived from the HBV Pre-S1 region described herein is 32-47 amino acid residues in length.In some particularly preferred embodiments, the exemplary amino acid sequence of the HBV entry inhibitory polypeptide derived from the HBV Pre-S 1 region is shown in SEQ ID NO: 9; the HBV entry inhibitory polypeptide derived from the HBV Pre-S 1 region also include the variants of the amino acid sequence shown in SEQ ID NO:9.

In some embodiments, the HBV entry-inhibiting polypeptide derived from the HBV Pre-S 1 region contains at least amino acid residues 1-32 of the exemplary sequence SEQ ID NO: 16 or variants thereof, preferably the HBV entry inhibitory polypeptide derived from the HBV Pre-S 1 region contains at least amino acid residues from position 1 to position 33, 34, 35, 36, 37, 38, 39, 40, 41, 42, 43, 44, 45 or 46 of the exemplary sequence SEQ ID NO: 16, or variants thereof.

In some embodiments, the HBV entry-inhibiting polypeptide derived from the HBV Pre-S 1 region refers to fragments of at least amino acid residues 13-44 at the N-terminal of the surface antigen pre-S1 region of HBV genotypes A, B, F, H, I, or variants thereof; fragments of at least amino acid residues 2-33 at the N-terminal of the pre-S1 region of the surface antigen of HBV genotype D, or variants thereof; or fragments of at least amino acid residues12-43 at the N-terminal of the pre-S1 region of the surface antigen of HBV genotype E, G, or variants thereof.

In some embodiments, the HBV entry-inhibiting polypeptide derived from the HBV Pre-S1 region contains fragments of at least amino acid residues 13-69 at the N-terminal of the pre-S 1 region of the surface antigen of HBV genotypes A, B, F, H, I, or variants thereof; fragments of at least amino acid residues 2-48 at the N-terminal of the pre-S 1 region of the surface antigen of HBV genotype D, or variants thereof, or fragments of at least amino acid residues 12-68 at the N-terminal of the pre S 1 region of the surface antigen of HBV genotype E, or G, or variants thereof.

As used herein, a "variant" related to an HBV entry-inhibiting polypeptide derived from the HBV Pre-S1 region refers to a difference in amino acid sequence from a given polypeptide, but retains the biological activity of inhibiting HBV entry or binding to NTCP. For example, in some embodiments, one or more of the following amino acid substitutions may be introduced into the derivative peptide derived from the amino acid sequence of positions 13-59 of the Pre-S1 region of HBV genotype C: N15D, F25L, G35K, N39E, F45L, N46K, N48H or N48Y or N48K, D50A, H51Q or HS 1N, E54K or E54D, A55S, N56K or N56D, and QS7K, exemplary sequences include but are not limited to those shown by SEQ ID NO: 15-32 sequence.

As used herein, the term "variant" also includes homologous polypeptide sequences found in different viral species, strains, or subtypes of the genus Hepatovirus. Based on the antigenic epitopes on its envelope protein, HBV is classified into four main serotypes (adr, adw, ayr, and ayw), and according to the variability of the nucleotide sequence in the genome, HBV is divided into nine genotypes (A, B, C, D, E, F, G, H and I). Thus, the term "variant" includes any of these homologous polypeptides found in HBV subtypes.

Herein, "variants" may also include polypeptides with natural flanking amino acid sequences from any of these HBV subtypes added at the N- and/or C-terminus, or variants thereof. For example, in some embodiments, a fragment comprising, but not limited to, at least 2-12 amino acid residues at the N-terminus of the pre-S1 region of the surface antigen of HBV genotypes A, B, F, H, I, or at least 2-11 amino acid residues at the N- terminus of the pre-S 1 region of the surface antigen of HBV genotypes E, G, serves as a naturally occurring flanking amino acid sequence for forming a fusion polypeptide with a peptide of amino acid 13-59 from the pre-S1 region of the C genotype surface antigen. For example, in some embodiments, the HBV entry-inhibiting polypeptide derived from the HBV Pre-S1 region described herein may contain an amino acid sequence selected from, but not limited to, any of SEQ ID NO: 2-32, and natural amino acid sequence flanking the N- and/or C-terminus derived from the surface antigen of any of HBV genotypes A-I. In some embodiments, the natural flanking amino acid sequences can be derived from the amino acid sequences of HBV surface antigens, including but not limited to GenBank accession numbers KC875260 (genotype A), AY220704 (genotype B), AF461363 (genotype C), AY796030 (amino acid sequence of HBV surface antigen of genotype D), AB205129 (genotype E), DQ823095 (genotype F), HE981176 (genotype G) or AB179747 (genotype H). For example, the HBV entry inhibitory polypeptides derived from the HBV Pre-S 1 region described herein may contain the amino acid sequences shown by SEQ ID NO: 9 and contain at its N and/or C terminus a natural flanking amino acid sequence from the pre-S1 region of the HBV genotype C. For example, the HBV entry inhibitory polypeptides derived from the HBV Pre-S1 region described herein may contain the amino acid sequences shown by SEQ ID NO: 9 and contain at its N and/or C terminus a natural flanking amino acid sequence from the pre-S1 region of any of the HBV genotype A, B, C, D, E, F, G and H. In some embodiments, the N-terminus and/or C-terminus of the HBV entry inhibiting polypeptide derived from the HBV Pre-S1 region described herein may independently contain a natural flanking amino acid sequence of 1-10, e.g., 1-8, 1-5, 1-3, (including all integers within these ranges) amino acid length. For example, the HBV entry inhibitory polypeptides derived from the HBV Pre-S1 region described herein may contain the amino acid sequences shown in SEQ ID NO: 9 and contain at its N-terminus a natural flanking amino acid sequence of 10 amino acids in length derived from the pre-S1 region of the HBV Genotype C. In other words, the HBV entry inhibitory polypeptide derived from the HBV Pre-S1 region may contain amino acids 2-59 of the pre-S1 region of HBV genotype C (SEQ ID NO: 4). Another example is that the HBV entry inhibitory polypeptides derived from the HBV Pre-S1 region described herein may contain the amino acid sequences shown in SEQ ID NO: 9 and contain at its N-terminus a natural flanking amino acid sequence of 9 amino acids in length derived from the pre-S1 region of the HBV Genotype E. In other words, the HBV entry inhibitory polypeptide derived from the HBV Pre-S 1 region may contain amino acids 13-59 of the pre-S1 region of HBV genotype C and amino acids 2-11 of the pre-S 1 region of HBV genotype E. It should be understood that any of the HBV entry inhibitory polypeptides derived from the HBV Pre-S 1 region may have a natural flanking amino acid sequence of any length extending from its N and/or C terminus, and the resulting HBV derived The HBV entry inhibiting polypeptide in the Pre-S1 region retains the biological activity of inhibiting HBV entry or binding to NTCP.

Herein, the "variant" related to the HBV entry-inhibiting polypeptide derived from the HBV Pre-S 1 region may also include one or more amino acid deletions, substitutions or insertions, while retaining the biological activity of inhibiting HBV entry or binding to NTCP. The HBV entry-inhibiting polypeptide derived from the HBV Pre-S1 region preferably retains the amino acid corresponding to the glycine at position 13 of the pre-S1 region of HBV genotype C (i.e., the N-terminal glycine of SEQ ID NO: 9). In some embodiments, the HBV entry inhibiting polypeptides derived from the HBV Pre-S1 region described herein may have one or more naturally occurring mutations in the pre-S1 region of HBV. For example, in some embodiments, relative to the sequence from the HBV pre-S1 region, the HBV entry inhibiting polypeptides derived from the HBV Pre-S1 region described herein may have 1-30, such as 1-20, 1-10, 1-8, 1-5 or 1-3 (including all integers within these ranges) amino acids deletions, substitutions or insertions. In some embodiments, the HBV entry inhibitory polypeptide derived from the HBV Pre-S1 region described herein may have 1-30, such as 1-20, 1-10, 1-8, 1-5 or 1-3 (including all integers within these ranges) amino acid deletions, substitutions or insertions relative to any amino acid sequence selected from SEQ ID NO: 2-32. In some embodiments, the HBV entry inhibitory polypeptide derived from the HBV Pre-S1 region described herein may have 1-30, such as 1-20, 1-10, 1-8, 1-5 or 1-3 (including all integers within these ranges) amino acid deletions, substitutions or insertions relative to any amino acid sequence selected from SEQ ID NO: 9-32. In some embodiments, the HBV entry inhibitory polypeptide derived from the HBV Pre-S 1 region described herein may have 1-3 amino acid deletions, substitutions, or insertions relative to the exemplary amino acid sequence of SEQ ID NO: 9. In some embodiments, the HBV entry inhibitory polypeptide derived from the HBV Pre-S1 region described herein may have 1-30, such as 1-20, 1-10, 1-8, 1-5 or 1-3 (including all integers within these ranges) amino acid deletions or insertions at the C-terminus relative to any amino acid sequence selected from SEQ ID NO: 2-32.

In various embodiments, the present description include, but are not limited to, the polypeptides and other polypeptides that has at least about 30%, 40%, 50%, 60%, 70%, 80%, 85%, 90%, 95%, 96%, 97%, 98% or 99% identity with any of the polypeptides. For example, the HBV entry inhibitory polypeptide derived from the HBV Pre-S1 region may comprise an amino acid sequence that has at least about 30%, 40%, 50%, 60%, 70%, 80%, 85%, 90%, 95%, 96%, 97%, 98% or 99% identity with any example sequence as shown by SEQ ID NO:2-32; preferably, the amino acid sequence having the identity is from any of the described HBV herein, including but not limited to HBV genotype A, B, C, D, E, F, G, H and I, more preferably from HBV genotype C. In some embodiments, the HBV entry inhibitory polypeptide derived from the HBV Pre-S 1 region can comprise an amino acid sequence that has at least about 30%, 40%, 50%, 60%, 70%, 80%, 85%, 90%, 95%, 96%, 97%, 98% or 99% identity with the example sequence as shown by SEQ ID NO:9; preferably, the amino acid sequence having the identity is from any of the described HBV herein, including but not limited to HBV genotype A, B, C, D, E, F, G, H and I, more preferably from HBV genotype C. The variants having a certain sequence identity with the HBV entry inhibitory polypeptides derived from the HBV Pre-S1 region described herein retain one or more biological activities of corresponding polypeptides, including inhibiting the biological activity of HBV virus entry or binding to NTCP active.

Herein, the N-terminus of the HBV entry inhibitory polypeptide derived from the HBV Pre-S1 region and variants thereof may be modified with a hydrophobic group, such as but not limited to myristic acid (myr), palmitic acid (plam), stearic acid (stearoyl), cholesterol (chol), oleic acid, linoleic acid, polyethylene glycol (PEG), arachidonic acid or other hydrophobic groups. In some embodiments, the hydrophobic group may be selected from myristic acid, palmitic acid, stearic acid, and cholesterol. In some embodiments, the hydrophobic group is myristic acid. In certain embodiments, the HBV entry inhibitory polypeptide derived from the HBV Pre-S 1 region described herein may comprise an amino acid sequence selected from any of SEQ ID NO: 2-32 or a variant thereof, wherein the N-terminus of the HBV entry inhibitory polypeptide derived from the HBV Pre-S1 region may be modified with a hydrophobic group selected from myristic acid, palmitic acid, stearic acid and cholesterol. In some embodiments, the polypeptide may comprise an amino acid sequence selected from any one of SEQ ID NO: 2-32 or a variant thereof, wherein the N-terminus of the HBV entry inhibitory polypeptide derived from the HBV Pre-S 1 region can be myristoylated. In some embodiments, the HBV entry inhibitory polypeptide derived from the HBV Pre-S 1 region described herein may comprise the amino acid sequence shown in SEQ ID NO: 9 or SEQ ID NO: 32, wherein the N-terminus of the HBV entry inhibitory polypeptide derived from the HBV Pre-S1 region may be myristoylated.

Herein, the C-terminus of the HBV entry inhibitory polypeptide derived from the HBV Pre-S1 region may be modified. C-terminus modifications may be selected from, but are not limited to, amidation (amination), prenylation, and other C-terminus modifications, or may be deleted. In some embodiments, the C-terminus modification may be amidation (NH₂). For example, the HBV entry inhibitory polypeptide derived from the HBV Pre-S 1 region may comprise the amino acid sequence as shown in SEQ ID NO: 9 or SEQ ID NO: 32, and its C-terminus may be amidated.

In some embodiments, the N-terminus of the HBV entry inhibitory polypeptide derived from the HBV Pre-S1 region may be modified with a hydrophobic group, and/or the C-terminus may be modified by other groups. In some embodiments, the N-terminus of the HBV entry inhibitory polypeptide derived from the HBV Pre-S 1 region may be modified with a hydrophobic group, and/or the C-terminus may be modified by amidation. In some embodiments, the HBV entry inhibitory polypeptide derived from the HBV Pre-S 1 region may comprise an amino acid sequence selected from any of SEQ ID NO: 2-32 and variants thereof, and its N-terminus being modified by hydrophobic group, and/or the C-terminus could be modified by amidation. For example, the HBV entry inhibitory polypeptide derived from the HBV Pre-S 1 region may comprise the amino acid sequence as shown in SEQ ID NO: 9 or SEQ ID NO: 32, and its N-terminus being modified by hydrophobic group of myristic acid (myr), palmitic acid (plam), stearic acid (stearoyl), cholesterol (chol), and its C-terminus being modified by amidation, such as the polypeptide shown by SEQ ID NO: 33-37. In some embodiments, the N-terminus of the HBV entry inhibitory polypeptide derived from the HBV Pre-S1 region may be modified with a hydrophobic group of myristic acid (myr), and/or the C-terminus can be modified by amidation. In certain embodiments, the polypeptide may comprise an amino acid sequence selected from any one of SEQ ID NO: 2-32 and variants thereof, the N-terminus of which may be modified by myristoylation, and/or the C-terminus may be modified by amidation. For example, the HBV entry inhibitory polypeptide derived from the HBV Pre-S1 region may comprise the polypeptide shown in SEQ ID NO: 9 or 32, the N-terminus of which is modified by a hydrophobic group of myristic acid (myr), and the C-terminus of which is modified by amidation, as shown in any of SEQ ID NO: 33, 34 and 38-44. In some embodiments of the invention, the HBV entry inhibitors include but are not limited to hepalatide (SEQ ID NO: 33), bulevirtide (SEQ ID NO: 43) and other HBV Pre-S 1 derived peptides. The name of hepalatide is N-myristoyl-glycyl-L-threonyl-L-asparaginyl-L-leucyl-L-seryl-L-valyl-L-proline acyl-L-asparaginyl-L-prolyl-L-leucyl-glycyl-L-phenylalanyl-L-phenylalanyl-L-prolyl-L-aspartyl -L-histidyl-L-glutamyl-L-leucyl-L-aspartyl-L-prolyl-L-alanyl-L-phenylalanyl-glycyl- L-alanyl-L-asparaginyl-L-seryl-L-asparaginyl-L-asparaginyl-L-prolyl-L-aspartyl-L-tryptophan acyl-L-aspartyl-L-phenylalanyl-L-asparaginyl-L-prolyl-L-asparaginyl-L-lysyl-L-aspartyl-L -histidyl-L-tryptophanyl-L-prolyl-L-glutamyl-L-alanyl-L-asparaginyl-L-glutamyl-L-valyl-glycine aminoamide. The name of Bulevirtide is N-myristoyl-glycyl-L-threonyl-L-asparaginyl-L-leucyl-L-seryl-L-valyl-L-proline acyl-L-asparaginyl-L-prolyl-L-leucyl-glycyl-L-phenylalanyl-L-phenylalanyl-L-prolyl-L-aspartyl -L-histidyl-L-glutamyl-L-leucyl-L-aspartyl-L-prolyl-L-alanyl-L-phenylalanyl-glycyl- L-alanyl-L-asparaginyl-L-seryl-L-asparaginyl-L-asparaginyl-L-prolyl-L-aspartyl-L-tryptophan acyl-L-aspartyl-L-phenylalanyl-L-asparaginyl-L-prolyl-L-asparaginyl-L-lysyl-L-aspartyl-L -histidyl-L-tryptophanyl-L-prolyl-L-glutamyl-L-alanyl-L-asparaginyl-L-lysyl-L-valyl-glycine aminoamide.

The sequences involved in this application are shown below.

| Number | Name | Amino acid sequence | Source |
|---|---|---|---|
| 1 | C1-119 | MGGWSSKPRQGMGTNLSVPNPLGFFPDHQLDPAFGANSN NPDWDFNPNKDHWPEANQVGAGAFGPGFTPPHGGLLGW SPQAQGILTTVPVAPPPASTNRQSGRQPTPISPPLRDSHPQA | Gene bank ID AF461363 |
| 2 | C2-119 | GGWSSKPRQGMGTNLSVPNPLGFFPDHQLDPAFGANSNNP DWDFNPNKDHWPEANQVGAGAFGPGFTPPHGGLLGWSP QAQGILTTVPVAPPPASTNRQSGRQPTPISPPLRDSHPQA | Genotype C Pre-S1(2-119) |
| 4 | C2-69 | GGWSSKPRQGMGTNLSVPNPLGFFPDHQLDPAFGANSNNP DWDFNPNKDHWPEANQVGAGAFGPGFTP | Genotype C Pre-S1(2-69) |
| 4 | C2-59 | GGWSSKPRQGMGTNLSVPNPLGFFPDHQLDPAFGANSNNP DWDFNPNKDHWPEANQVG | Genotype C Pre-S1(2-59) |
| 5 | C13-119 | GTNLSVPNPLGFFPDHQLDPAFGANSNNPDWDFNPNKDH WPEANQVGAGAFGPGFTPPHGGLLGWSPQAQGILTTVPVA PPPASTNRQSGRQPTPISPPLRDSHPQA | Genotype C Pre-S1(13-119) |
| 6 | C13-88 | GTNLSVPNPLGFFPDHQLDPAFGANSNNPDWDFNPNKDH WPEANQVGAGAFGPGFTPPHGGLLGWSPQAQGILTTV | Genotype C Pre-S1(13-88) |
| 7 | C13-72 | GTNLSVPNPLGFFPDHQLDPAFGANSNNPDWDFNPNKDH WPEANQVGAGAFGPGFTPPHG | Genotype C Pre-S1(13-72) |
| 8 | C13-67 | GTNLSVPNPLGFFPDHQLDPAFGANSNNPDWDFNPNKDH WPEANQVGAGAFGPGF | Genotype C Pre-S1(13-67) |
| 9 | C13-59 | GTNLSVPNPLGFFPDHQLDPAFGANSNNPDWDFNPNKDH WPEANQVG | Genotype C Pre-S1(13-59) |
| 10 | C13-52 | GTNLSVPNPLGFFPDHQLDPAFGANSNNPDWDFNPNKDH W | Genotype C Pre-S1(13-52) |
| 11 | C13-47 | GTNLSVPNPLGFFPDHQLDPAFGANSNNPDWDFNP | Genotype C Pre-S1(13-47) |
| 12 | C13-42 | GTNLSVPNPLGFFPDHQLDPAFGANSNNPD | Genotype C Pre-S1(13-42) |
| 13 | C13-37 | GTNLSVPNPLGFFPDHQLDPAFGAN | Genotype C Pre-S1(13-37) |
| 14 | C13-32 | GTNLSVPNPLGFFPDHQLDP | Genotype C Pre-S1(13-32) |
| 15 | C13-59 (N15D) | GTDLSVPNPLGFFPDHQLDPAFGANSNNPDWDFNPNKDH WPEANQVG | Genotype C Pre-S1(13-59) |
| 16 | C13-59 | GTNLSVPNPLGFLPDHQLDPAFGANSNNPDWDFNPNKDH | Genotype C Pre- |
| | (F25L) | WPEANQVG | S1(13-59) |
| 17 | C13-59 (G35K) | GTNLSVPNPLGFFPDHQLDPAFKANSNNPDWDFNPNKDH WPEANQVG | Genotype C Pre-S1(13-59) |
| 18 | C13-59 (N39E) | GTNLSVPNPLGFFPDHQLDPAFGANSENPDWDFNPNKDH WPEANQVG | Genotype C Pre-S1(13-59) |
| 19 | C13-59 (N46K) | GTNLSVPNPLGFFPDHQLDPAFGANSNNPDWDFKPNKDH WPEANQVG | Genotype C Pre-S1(13-59) |
| 20 | C13-59 (F45L) | GTNLSVPNPLGFFPDHQLDPAFGANSNNPDWDLNPNKDH WPEANQVG | Genotype C Pre-S1(13-59) |
| 21 | C13-59 (N48H) | GTNLSVPNPLGFFPDHQLDPAFGANSNNPDWDFNPHKDH WPEANQVG | Genotype C Pre-S1(13-59) |
| 22 | C13-59 (N48Y) | GTNLSVPNPLGFFPDHQLDPAFGANSNNPDWDFNPYKDH WPEANQVG | Genotype C Pre-S1(13-59) |
| 23 | C13-59 (N48K) | GTNLSVPNPLGFFPDHQLDPAFGANSNNPDWDFNPKKDH WPEANQVG | Genotype C Pre-S1(13-59) |
| 24 | C13-59 (D50A) | GTNLSVPNPLGFFPDHQLDPAFGANSNNPDWDFNPNKAH WPEANQVG | Genotype C Pre-S1(13-59) |
| 25 | C13-59 (H51Q) | GTNLSVPNPLGFFPDHQLDPAFGANSNNPDWDFNPNKDQ WPEANQVG | Genotype C Pre-S1(13-59) |
| 26 | C13-59 (H51N) | GTNLSVPNPLGFFPDHQLDPAFGANSNNPDWDFNPNKDN WPEANQVG | Genotype C Pre-S1(13-59) |
| 27 | C13-59 (E54K) | GTNLSVPNPLGFFPDHQLDPAFGANSNNPDWDFNPNKDH WPKANQVG | Genotype C Pre-S1(13-59) |
| 28 | C13-59 (E54D) | GTNLSVPNPLGFFPDHQLDPAFGANSNNPDWDFNPNKDH WPDANQVG | Genotype C Pre-S1(13-59) |
| 29 | C13-59 (A55S) | GTNLSVPNPLGFFPDHQLDPAFGANSNNPDWDFNPNKDH WPESNQVG | Genotype C Pre-S1(13-59) |
| 30 | C13-59 (N56K) | GTNLSVPNPLGFFPDHQLDPAFGANSNNPDWDFNPNKDH WPEAKQVG | Genotype C Pre-S1(13-59) |
| 31 | C13-59 (N56D) | GTNLSVPNPLGFFPDHQLDPAFGANSNNPDWDFNPNKDH WPEADQVG | Genotype C Pre-S1(13-59) |
| 32 | C13-59 (Q57K) | GTNLSVPNPLGFFPDHQLDPAFGANSNNPDWDFNPNKDH WPEANKVG | Genotype C Pre-S1(13-59) |

| Number | Name | N- terminal modific ation | Amino acid sequence | C- terminal modific ation | Sourse |
|---|---|---|---|---|---|
| 33 | myrC13-59 NH2 | Myr | GTNLSVPNPLGFFPDHQLDPAFGANSN NPDWDFNPNKDHWPEANQVG | NH2 | Genotype C Pre-S1(13-59) |
| 34 | MyrC13-59 NH2 (Q57K) | Myr | GTNLSVPNPLGFFPDHQLDPAFGANSN NPDWDFNPNKDHWPEANKVG | NH2 | Genotype C Pre-S1(13-59) |
| 35 | plam C13-59 NH2 | Plam | GTNLSVPNPLGFFPDHQLDPAFGANSN NPDWDFNPNKDHWPEANQVG | NH2 | Genotype C Pre-S1(13-59) |
| 36 | stea C13-59 NH2 | Stearoyl | GTNLSVPNPLGFFPDHQLDPAFGANSN NPDWDFNPNKDHWPEANQVG | NH2 | Genotype C Pre-S1(13-59) |
| 37 | chol C13-59 NH2 | Chol | GTNLSVPNPLGFFPDHQLDPAFGANSN NPDWDFNPNKDHWPEANQVG | NH2 | Genotype C Pre-S1(13-59) |
| 38 | MyrA13-59 NH2 | Myr | GTNLSVPNPLGFFPDHQLDPAFGANSN NPDWDFNPVKDDWPAANQVG | NH2 | Genotype A Pre-S1(13-59) |
| 39 | MyrB13-59 NH2 | Myr | GTNLSVPNPLGFFPDHQLDPAFKANSE NPDWDLNPNKDNWPDANKVG | NH2 | Genotype B Pre-S1(13-59) |
| 40 | MyrD2-48 NH2 | Myr | GQNLSTSNPLGFFPDHQLDPAFRANTA NPDWDFNPNKDTWPDANKVG | NH2 | Genotype D Pre-S 1 (2-48) |
| 41 | MyrE12-58 NH2 | Myr | GKNISTTNPLGFFPDHQLDPAFRANTR NPDWDHNPNKDHWTEANKVG | NH2 | Genotype E Pre-S1(12-58) |
| 42 | MyrF13-59 NH2 | Myr | GQNL SVPNPL GFFPDHQLDPLFRANSS SPDWDFNTNKDSWPMANKVG | NH2 | Genotype F Pre-S1(13-59) |
| 43 | MyrG12-58 NH2 | Myr | GKNLSASNPLGFLPDHQLDPAFRANTN NPDWDFNPKKDPWPEANKVG | NH2 | Genotype G Pre-S1(12-58) |
| 44 | MyrH13-59 NH2 | Myr | GQNLSVPNPLGFFPDHQLDPLFRANSS SPDWDFNTNKDNWPMANKVG | NH2 | Genotype H Pre-S1(13-59) |

In one embodiment of the present description, hepalatide or bulevirtide treatment is administered based on NAs treatment. In a further preferred scheme, hepalatide treatment is administered in addition to NAs treatment.

In one embodiment, the therapeutic dose of hepalatide is 2.1-10.5 mg per day. In a preferred embodiment, the therapeutic dose of hepalatide is 4.2-8.4 mg per day. In a further preferred embodiment, the therapeutic dose of hepalatide is 4.2 mg per day.

In one embodiment, the treatment period of hepalatide is 1-96 weeks. In a preferred embodiment, the treatment period of hepalatide is 12-48 weeks. In a further preferred embodiment, the treatment period of hepalatide is 24-48 weeks. In a more preferred embodiment, the treatment period of hepalatide is 48 weeks.

Herein, the anti-Pre-S 1 antibody includes but not limited to anti-HBV Pre-S 1 monoclonal antibody, anti-HBV Pre-S1 bifunctional antibody (double antibody), anti-HBV Pre-S1 polyclonal antibody (polyclonal antibody), anti-HBV Pre-S1 serum, and other forms of antibodies and compositions thereof, which can bind to HBV Pre-S1 to inhibit HBV infection.

Herein, the anti-surface antigen antibodies include but not limited to anti-surface antigen monoclonal antibodies, anti-surface antigen diabodies (diabodies), anti-surface antigen polyclonal antibodies (polyclonal antibodies), anti-surface antigen serum, HBIG and other forms of antibodies and compositions thereof, which can bind to surface antigens to inhibit HBV infection.

Herein, the other agents that can inhibit HBV from entering liver cells or infecting liver cells include but are not limited to small molecules, proteins and other molecules, which can act on NTCP, HBV virus or other targets to achieve the technical effect of inhibiting HBV infection.

Herein, the NAs are treated with an HBV entry inhibitor for a certain period of combined treatment, in one embodiment, for 1-96 weeks of combined treatment. In a preferred embodiment, the combined treatment is for 12-60 weeks. In a further preferred embodiment, the combined treatment is for 24-48 weeks. In a still further preferred embodiment, the combined treatment is for 48 weeks.

Herein, the non-rebound of HBV in CHB patients after the termination of treatment refers to the non-rebound of HBV within at least 24 weeks after the patient stops the combined treatment. In a preferred embodiment, the non-rebound of HBV in CHB patients after the termination of treatment refers to the non-rebound of HBV within at least 48 weeks after the patient stops the combined treatment. In a further preferred embodiment, the non-rebound of HBV in CHB patients after the termination of treatment refers to the non-rebound of HBV within at least 96 weeks after the patient stops the combined treatment.

In some embodiments, the non-rebound of HBV in CHB patients after the termination of treatment refers to the success of medicine withdrawal after the patient stops the combined treatment, and the patient does not need to receive NAs treatment again (retreatment). In a preferred embodiment, the non-rebound of HBV in CHB patients after the termination of treatment refers to the HBV DNA being continuously lower than 2000 IU/ml after the patient stops the combined treatment. In a preferred embodiment, the non-rebound of HBV in CHB patients after the termination of treatment means that the HBV DNA continues to be lower than the lower limit of quantitative detection (less than 20 IU/ml) after stopping the combined treatment. In a preferred embodiment, the non-rebound of HBV in CHB patients after the termination of treatment means that the HBV DNA continues to be undetectable (less than 10 IU/ml) after the patient stops the combined treatment.

In one embodiment of the present description, for CHB patients receiving continuous NAs treatment, the basic NAs are changed to TDF or TAF, and HBV entry inhibitors are administered for combined treatment. In a further preferred embodiment, CHB patients change the basic NAs to TAF, and HBV entry inhibitors are administered for combined treatment.

In one embodiment of the present description, in addition to continuous NAs treatment, CHB patients are administered by HBV entry inhibitors and immunomodulators for combined treatment, and the immunomodulators include but not limited to interferon (IFN), toll-like receptor (Toll-like receptors, TLR) agonist, CPG (Cytidine-phosphatte-guanonine), CPG ODN (CpG oligonucleotide), PD-1 (Programmed Death Receptor-1) inhibitor, PD-L1 (Programmed Death Receptor ligan 1) inhibitors, interleukins (IL), cytokines, and other immunomodulators. The TLR agonists include but are not limited to TLR1, TLR2, TLR3, TLR4, TLR5, TLR6, TLR7, TLR8, TLR9, TLR10, TLR11 and other TLR receptor agonists. Among them, PD-1 inhibitors include but are not limited to anti-PD-1 monoclonal antibodies, anti-PD-1 bispecific antibodies, anti-PD-1 polyclonal antibodies, small molecule medicines that inhibit PD-1 or PD-1 signaling, and others PD-1 inhibitors. Among them, PD-L1 inhibitors include but are not limited to anti-PD-L1 monoclonal antibody, anti-PD-L1 bispecific antibody, anti-PD-L1 polyclonal antibody, anti-PD-L1, recombinant PD-L1 protein, recombinant PD-L1 fusion proteins, and small molecule medicines that inhibit PD-L1 or PD-L1 signaling and other PD-L1 inhibitors.

In one embodiment of the present description, the administered HBV entry inhibitors include but are not limited to HBV Pre-S 1 derived peptides, anti-Pre-S 1 antibodies, anti-surface antigen antibodies or other agents that can inhibit HBV from entering liver cells or infecting liver cells. The HBV Pre-S1 derived peptides include but not limited to hepalatide (SEQ ID NO: 33), bulevirtide (SEQ ID NO: 34) and other HBV Pre-S1 derived peptides. The other HBV Pre-S1 derived peptides include but are not limited to the exemplified sequences shown in SEQ ID NO: 2-44 and other polypeptides derived from the Pre-S1 region of HBV genotype A-I, wherein the peptides can bind to NTCP and inhibit HBV infection.

In one embodiment, hepalatide or bulevirtide treatment is administered in addition to NAs treatment. In a further preferred scheme, hepalatide treatment is administered in addition to NAs treatment. In one embodiment, the therapeutic dose of hepalatide is 2.1-10.5 mg per day. In one preferably embodiment, the therapeutic dose of hepalatide is 4.2-8.4 mg per day. In a further preferred embodiment, the therapeutic dose of hepalatide is 4.2 mg per day. In one embodiment, the treatment period of hepalatide is 1-96 weeks. In a preferred embodiment, the treatment period of hepalatide is 12-48 weeks. In a further preferred embodiment, the treatment period of hepalatide is 24-48 weeks. In a more preferred embodiment, the treatment period of hepalatide is 48 weeks. In some embodiments of the invention, the administered immunomodulator is interferon (IFN). In some embodiments of the invention, the administered interferon is IFN-α, IFN-β, IFN-γ. In a preferred embodiment, the administered interferon is IFN α-2a or IFN α-2b. In one embodiment of the present description, the administered interferon is common interferon or peginterferon. In a preferred embodiment, the administered interferon is polyethylene glycol (PEG) interferon. In a further preferred embodiment, the administered interferon is PEG IFN α-2a or PEG IFN α-2b, and these interferons include but are not limited to Pegasys, Pegabine, PegIntron and other interferons. Preferably, the dose of interferon is lower than the standard dose for clinical treatment. In one embodiment of the present description, the administered interferon is Pegasys, and its dose is 1-360 ug per week; in one embodiment, its dose is 30-180 ug per week; in one embodiment, its dose is 60-135 ug per week; in one embodiment, the dose is 90 ug per week. The combined treatment of NAs and the HBV entry inhibitor are administered for a certain period together with interferon, in one embodiment, the combined treatment is 1-96 weeks. In a preferred embodiment, the combined treatment is for 12-60 weeks. In a further preferred embodiment, the combined treatment is for 24-48 weeks. In a still further preferred embodiment, the combined treatment is for 48 weeks.

In the method for limiting NAs treatment provided by the present description, basic NAs treatment includes but not limited to lamivudine, telbivudine, adefovir dipivoxil, tenofovir, entecavir, tenofovir alafenamide hemifumarate and other NAs medicines; HBV entry blockers (inhibitors) include but not limited to hepalatide, bulevirtide and other HBV entry blockers; immunemodulators include but not limited to interferon and other immunomodulators. The limited NAs treatment method provided by the present description at least comprises administering HBV entry blocker treatment in addition to NAs treatment, and can also further comprise administering immunomodulator treatment. The methods for limiting NAs treatment provided by the present description include but are not limited to the schemes listed in the following table. In some embodiments of the present description, schemes 6, 13, 27, 34 may be employed, but not limited to these schemes.

| Scheme number | Basic NAs treatment | Treatment | |
|---|---|---|---|
| | | HBV entry inhibitor | Immunomodulators |
| 1 | Lamivudine | Hepalatide | None |
| 2 | Adefovir dipivoxil | Hepalatide | None |
| 3 | Telbivudine | hepalatide | None |
| 4 | Entecavir | Hepalatide | None |
| 5 | Tenofovir | Hepalatide | None |
| 6 | Tenofovir alafenamide hemifumarate | Hepalatide | None |
| 7 | Other NAs | Hepalatide | None |
| 8 | Lamivudine | Bulevirtide | None |
| 9 | Adefovir dipivoxil | Bulevirtide | None |
| 10 | Telbivudine | Bulevirtide | None |
| 11 | Entecavir | Bulevirtide | None |
| 12 | Tenofovir | Bulevirtide | None |
| 13 | Tenofovir alafenamide hemifumarate | Bulevirtide | None |
| 14 | Other NAs | Bulevirtide | None |
| 15 | Lamivudine | Other HBV entry blocker | None |
| 16 | Adefovir dipivoxil | Other HBV entry blocker | None |
| 17 | Telbivudine | Other HBV entry blocker | None |
| 18 | Entecavir | Other HBV entry blocker | None |
| 19 | Tenofovir | Other HBV entry blocker | None |
| 20 | Tenofovir alafenamide hemifumarate | Other HBV entry blocker | None |
| 21 | Other NAs | Other HBV entry blocker | None |
| 22 | Lamivudine | Hepalatide | Interferon |
| 23 | Adefovir dipivoxil | Hepalatide | Interferon |
| 24 | Telbivudine | Hepalatide | Interferon |
| 25 | Entecavir | Hepalatide | Interferon |
| 26 | Tenofovir | Hepalatide | Interferon |
| 27 | Tenofovir alafenamide hemifumarate | Hepalatide | Interferon |
| 28 | Other NAs | Hepalatide | Interferon |
| 29 | Lamivudine | Bulevirtide | Interferon |
| 30 | Adefovir dipivoxil | Bulevirtide | Interferon |
| 31 | Telbivudine | Bulevirtide | Interferon |
| 32 | Entecavir | Bulevirtide | Interferon |
| 33 | Tenofovir | Bulevirtide | Interferon |
| 34 | Tenofovir alafenamide hemifumarate | Bulevirtide | Interferon |
| 35 | Other NAs | Bulevirtide | Interferon |
| 36 | Lamivudine | Hepalatide | Other immunomodulators |
| 37 | Adefovir dipivoxil | Hepalatide | Other immunomodulators |
| 38 | Telbivudine | Hepalatide | Other immunomodulators |
| 39 | Entecavir | Hepalatide | Other immunomodulators |
| 40 | Tenofovir | Hepalatide | Other immunomodulators |
| 41 | Tenofovir alafenamide hemifumarate | Hepalatide | Other immunomodulators |
| 42 | Other NAs | Hepalatide | Other immunomodulators |
| 43 | Tenofovir alafenamide hemifumarate | Other HBV entry inhibitor | Interferon |
| 44 | Tenofovir alafenamide hemifumarate | Other HBV entry inhibitor | Other immunomodulators |
| 45 | Other NAs | Other HBV entry inhibitor | Interferon |
| 46 | Other NAs | Other HBV entry inhibitor | Other immunomodulators |

In some embodiments, the present description provides the use of an HBV virus entry inhibitor and an optional immunomodulator in the preparation of a medicament or kit for limiting or terminating nucleoside (acid) drug treatment of chronic hepatitis B. The HBV virus entry inhibitors, immunomodulators, nucleoside (acid) medicines, medicaments and kits are as described in any of the above embodiments. In some embodiments, the present description provides the HBV virus entry inhibitor and the optional immunomodulator described in any embodiment herein or a medicament or a kit containing the HBV virus entry inhibitor and optionally immunomodulator according to any embodiment. In some embodiments, the medicament or kit of the present description is used in the method of limiting or terminating NAs medicine treatment of chronic hepatitis B as described in any of the embodiments herein. In some embodiments, the kit of the present description further comprises one or more doses of nucleoside (acid) medicines described in any embodiment herein. Preferably, the pharmaceutical preparations contained in the kit meet the dosage required for one or more courses of treatment described herein.

The disclosure if further illustrated by the specific examples described below. It should be understood that these examples are merely illustrative, and do not limit the scope of the present disclosure. For the experimental methods without specific conditions indicated in the following examples, the conventional conditions or the conditions suggested by the manufacturer are usually followed. Percentages and parts are by weight unless otherwise indicated.

### Example 1: Randomized double-blind placebo-controlled multicenter phase II clinical trial protocol of TAF combined with PEG interferon and hepalatide for a limited course of treatment of chronic hepatitis B (L47-HB-FIN-1)

### Number of subjects

It is planned to enroll 30 cases.

### Purpose

Main purpose: CHB patients with NA treatment who have been treated for a limited course of treatment with TAF combined with PEG interferon and hepalatide, to achieve a degree of cure or clinical cure after medicine withdrawal.

Secondary objectives: 1) To study the success rate of terminating NA treatment by a limited course of treatment of TAF combined with PEG interferon and hepalatide; 2) To study the safety of a limited course of treatment of TAF combined with PEG interferon and hepalatide.

### Test design

This study is a two-arm parallel design, randomized, double-blind, placebo-controlled, multicenter clinical trial. The overall design is shown in Figure 1. CHB patients who had been treated with NA for at least 2 years and met the inclusion and exclusion criteria were randomized into the hepalatide group and the placebo group at a ratio of 1:1, with 15 subjects in each group (see Table 1). Both groups were given TAF (25 mg/day) and PEG interferon (90 ug/week), and hepalatide (4.2 mg/day) and placebo, respectively, for 48 weeks of continuous treatment. At the end of treatment, all medicines were terminated, and the primary endpoint was evaluated 24 weeks after medicine withdrawal, and follow-up visits were conducted.

**Table 1: Experimental groups**

| Groups | Dosage | Number | Basic treatment | |
|---|---|---|---|---|
| | (s.c.QD) | | (p.o.QD) | (s.c.QW) |
| L47 placebo group | 0 mg/day | 15 | TAF (25 mg/day) | Pegasys (90 ug/week) |
| L47 experimental medicine group | 4.2 mg/day | 15 | | |

### Inclusion Criteria

1) 18 years old ≤ age ≤ 60 years old, both male and female;
2. HBsAg (+) or HBV DNA (+) ≥ 6 months (diagnosed as "chronic hepatitis B" by the investigator);
3. HBeAg (-);
4. Received NAs stable treatment for ≥ 2 years;
5. HBV DNA < LLQD (lower limit of quantitative detection) at the time of screening;
6. ULN<ALT<10xULN;
7. Serum total bilirubin <2×ULN;
8. No birth plan within two years, agree to take effective contraceptive measures during the entire treatment period and within 3 months after the last dose, and the female is not pregnant or breastfeeding;
9. Has not been used as a subject of other clinical trials within 3 months;
10. Good compliance with the research protocol;
11. The subject understands and agrees to sign the informed consent form.

### Exclusion criteria

1. Pegylated interferon treatment contraindications, such as severe depression, epilepsy, autoimmune diseases, uncontrolled thyroid dysfunction, etc.
2. There is clinical evidence of liver cirrhosis: such as abdominal color Doppler ultrasound, CT and other imaging examinations clearly show liver cirrhosis; liver biopsy Metavir fibrosis score is 4 points; the investigator clinically diagnoses liver cirrhosis;
3. Decompensated liver disease: prothrombin time>1.2×ULN, serum albumin<35 g/L;
4. Liver function Child-Pugh grade B-C or score > 6 points;
5. Those who have any of the following conditions:
   1) History of decompensated liver disease (ascites, hepatic encephalopathy, variceal hemorrhage, hepatorenal syndrome, etc.);
   2) History of severe heart disease (including unstable or uncontrolled heart disease within 6 months);
   3) Uncontrolled epilepsy, severe mental illness or history of serious mental illness;
   4) History of organ transplantation;
   5) Uncontrolled diabetes and hypertension;
   6) Those with autoimmune diseases, immune-related extrahepatic manifestations (vasculitis, purpura, nodular arteritis, peripheral neuropathy, and glomerulonephritis), thyroid disease, malignant tumor, and immunosuppressive therapy;
   7) Accompanied by basic diseases such as malignant tumors, severe infections, heart failure and chronic obstructive pulmonary disease, and other serious diseases;
   8) History of alcohol or drug abuse.
6. Creatinine clearance rate <60 mL/min;
7. Combined with hepatitis A, C, D, E virus infection, HIV infection;
8. Subjects who must be treated with other anti-HBV nucleoside (acid) medicines except TAF during the treatment period;
9. Subjects who have used interferon within 6 months before the screening period;
10. Anti-HBV Pre-S1 antibody positive;
11. Abnormal blood routine examination: white blood cell count<3×10⁹/L, neutrophil count<1.5×10⁹/L, platelets<60×10⁹/L.
12. Female pregnancy test positive;
13. Those who are taking prohibited medicines in this study and cannot stop the medicines;
14. Patients who are known to be allergic to the experimental medicines or basic treatment medicines;
15. Those who are obviously abnormal in other laboratory or auxiliary examinations are not suitable for participating in this experiment.

### Medicine

1. Tenofovir Alafenamide Tablets (TAF)
   Dosage form: tablet;
   Specification: 25 mg;
   Transportation and storage: Store below 30°C;
   Validity period: 24 months;
   Approval number: Import drug registration No. H20180060;
   Manufacturer: Patheon Inc.
2. Pegylated interferon (Pegasys)
   Dosage form: injection;
   Specification: 180 µg/0.5 ml;
   Transportation and storage: sealed, protected from light, stored in the original packaging at 2-8°C; do not freeze;
   Validity period: 24 months;
   Approval number: NMPA Approval No. J20120075;
   Manufacturer: Shanghai Roche Pharmaceutical Co., Ltd.
3. Hepalatide for Injection
   Dosage form: powder injection;
   Specification: 2.1 mg;
   Transportation and storage: transportation temperature ≤ 8 °C, 2-8 °C sealed and protected from light;
   Validity period: 36 months;
   Manufacturer: Hainan Shuangcheng Pharmaceutical Co., Ltd.
4. Hepalatide placebo
   Dosage form: powder injection;
   Specification: 0 mg;
   Transportation and storage: transportation temperature ≤ 8 °C, 2-8 °C sealed and protected from light;
   Validity period: 36 months;
   Manufacturer: Hainan Shuangcheng Pharmaceutical Co., Ltd.

### Basic treatment

1. TAF (25 mg/day, p.o. QD), continuous treatment for 48 weeks.
2. Pegasys (90 µg/0.25 ml, s.c. QW), continuous treatment for 48 weeks.

### experimental treatment

Hepalatide treatment (4.2 mg, s.c. QD) was given in addition to the basic treatment for 48 weeks of continuous treatment.

There is no requirement for the administration time of hepalatide, but it is recommended to inject subcutaneously 30 minutes before bedtime after dinner.

### control treatment

Subcutaneous injection of hepalatide placebo once a day was given in addition to the basic treatment, and the treatment continued for 48 weeks.

### Expected treatment

Treatment period: 48 weeks;
Follow-up period: 24 weeks.

### study endpoint

### primary endpoint

1) Virological sustained response: 24 weeks after medicine withdrawal, serum HBV DNA <20 IU/mL (72 W);
2) HBsAg turned negative (72 W).

### secondary endpoint

1) NA retreatment rate (72 W);
2) Virological recurrence (72 W);
3) Clinical recurrence (72 W);
4) HBsAg decreased compared with baseline (72 W);
5) HBsAg seroconversion (72 W);
6) Changes in liver elasticity compared with baseline (72 W).

### Effectiveness index

### Effectiveness index:

HBV DNA, HBsAg, HBsAb, HBeAb, ALT, LSM, HBcrAg, HBV pgRNA

### Validity definition:

Virological response: during treatment, serum HBV DNA <20 IU/mL (lower limit of detection);
Virological sustained response: response at the end of treatment and follow-up for 24 or 48 weeks after medicine withdrawal;
Virological breakthrough: for patients with good compliance with nucleoside (acid) analog therapy, without changing the treatment, the HBV DNA level increased by >1 logic₁₀ IU/mL from the lowest value in the treatment, or turned negative and then turned positive, which is confirmed by repeating the test with identical reagent after 1 month, with or without ALT elevation;
Virological recurrence: response at the end of treatment has been achieved, but the HBV DNA levels in two times of central laboratory tests 1 month after medicine withdrawal are both > 2000 IU/ml;
Clinical relapse: ALT>2×ULN, HBV DNA>2000 IU/ml in patients who have achieved sustained virological response after stopping treatment;
HBsAg negative conversion: HBsAg turned to negative;
HBsAg seroconversion: HBsAg turned to negative with HBsAb positive;
HBeAg seroconversion: HBeAg turned to negative with HBeAb positive;

### Safety Index

Vital signs, physical examination;
Laboratory tests: routine blood test and routine urine test, blood biochemistry, coagulation, thyroid function, alpha-fetoprotein;
Auxiliary examination: electrocardiogram, B-ultrasound examination (liver, gallbladder, spleen, pancreas, kidney, ascites), liver CT plain scan;
AE targeted inspection;
Key indicators: hematology, liver function, blood sugar, blood lipids, blood cholesterol, total bile acids, blood phosphorus, blood fibrinogen.

### Safety Evaluation

Symptomatic adverse events are classified into mild (transient symptoms, which do not affect the subjects' daily activities), moderate (obvious symptoms, which have a certain impact on the subjects' daily activities) and severe (significantly affect the subjects' daily activities, unacceptable). Objective adverse events were graded according to NCI CTCAE v5.0, using 5-level criteria.

Indications for antiviral retreatment (meet one of the following).
1. With an interval of 1 mouth, two consecutive central laboratory test results confirmed: HBV DNA>2000 IU/mL, ALT>2×ULN;
2. Two consecutive visiting clinical center test results confirmed: direct bilirubin increased by > 1.5 mg/dL (25 µmol/L) compared with the baseline, and ALT > 1 × ULN;
3. Two consecutive visit results confirmed: prothrombin time prolongation ≥ 2 seconds or INR ≥ 0.5, and ALT > 1 × ULN;
4. Two consecutive central laboratory test results confirmed: ALT>10×ULN, with or without clinical symptoms, and liver function damage caused by other reasons is excluded;
5. 5×ULN<ALT<10×ULN for ≥4 weeks, and liver function damage caused by other reasons is excluded;
6. 2×ULN<ALT<5×ULN for ≥12 weeks, HBV DNA>LLQD, and liver function damage caused by other reasons is excluded;
7. If the patient has elevated ALT or HBV DNA, but does not meet one of the above criteria, the investigator will decide whether to restart the antiviral treatment, or continue the scheduled visit/additional visit, or the patient should be withdrawed from the study.

### anti-Pre-S1 antibody

Blood samples were collected at baseline and at weeks 4, 12, 24, 48, and 72 during treatment to detect anti-Pre-S 1 antibodies.

### Trial Endpoint

All enrolled subjects completed 48 weeks of treatment and 24 weeks of follow-up. If there is a dropout or loss of follow-up, no additions will be made.

### Follow-up

After completing the 72-week trial, follow-up were continued every 6 months for 3 years.

### Statistical methods

Analysis of the primary endpoint: mITT (main analysis) and PPS were used to test the primary efficacy endpoint, and multiple comparison factors were not considered for the statistics of different primary endpoints. The primary end point of the L47 4.2 mg dose group and the placebo group were subjected to a two-sided test between groups. The test level was set at 0.025 (one-sided), and the confidence interval method was used to judge superiority. If the lower limit of the one-sided 97.5% confidence interval of the difference between groups is greater than 0, it can be considered that the L47 4.2 mg dose group test medicine is better than the placebo.

Secondary endpoint analysis: the secondary efficacy endpoints were tested by mITT and PPS respectively. The L47 4.2 mg dose group was compared with the placebo group for the secondary endpoint, and the test level was one-sided 0.025.

Analysis of other endpoint indicators: according to the characteristics of the data, parametric or non-parametric tests were used to compare the differences between groups.

Safety analysis: a safety set (SS) was used, comprising all subjects exposed to at least one dose of the trial medicine (L47, L47 placebo). Descriptive statistics were used for safety indicators.

For subjects who have HBV virological breakthroughs or ALT SAEs that require emergency unblinding during the trial, the efficacy endpoint is treatment failure.

## Claims

1. Use of an HBV virus entry inhibitor and an optional immunomodulator in the preparation of a medicament or kit for limiting or terminating nucleoside (acid) medicine treatment of chronic hepatitis B.

2. The use according to claim 1, wherein the HBV virus entry inhibitor is selected from the group consisting of: HBV Pre-S1 derived peptides or HBV entry inhibitory polypeptides derived from HBV Pre-S 1 regions, anti-Pre-S 1 antibodies, anti-surface antigen antibodies or other agents capable of inhibiting HBV from entering liver cells or infecting liver cells; preferably, the HBV entry inhibiting polypeptide derived from the HBV Pre-S 1 region is derived from the pre-S 1 region of the surface antigen of any one of HBV genotypes A, B, C, D, E, F, G, H and I; preferably, the length of the HBV entry inhibitory polypeptide derived from the HBV Pre-S1 region is 10-118 amino acid residues;
the immunomodulator comprises interferon, toll-like receptor agonist, CPG, CPG ODN, PD-1 inhibitor, PD-L1 inhibitor, interleukin and cytokine; preferably, the interferon is selected from IFN-kinesin, IFN-kinesin and IFN-kinesin, preferably IFN-kinesin cells and/or IFN-kinesin cells, more preferably PEG IFNα-2a and/or PEG IFNα-2b.

3. The use according to claim 2, wherein,
the HBV entry inhibitory polypeptide derived from the HBV Pre-S1 region is derived from the pre-S 1 region of the surface antigen of HBV genotype C ; preferably, the HBV entry inhibitory polypeptide is the fragment of N-terminus of the pre-S1 region of the surface antigen of HBV genotype C, or variants thereof, the fragment at least comprises the amino acid s13-44 of the N-terminus; preferably, the HBV entry inhibitory polypeptide contains the amino acid residue of amino acids 2-119, 2-69, 2-59, 13-119, 13-88, 13-72, 13-67, 13-59, 13-52 or 13-47 of the pre-S1 region of the surface antigen of HBV genotype C, or variants thereof; or
the HBV entry inhibitory polypeptide derived from the HBV Pre-S1 region is a fragment of the N-terminus of the pre-S1 region of the surface antigen of HBV genotype A, B, F, H or I, or a variant thereof, and the fragment comprises at least the amino acid 13-44 of the N-terminus, preferably comprises at least the amino acid 13-69 of the N-terminus; or
the HBV entry inhibitory polypeptide derived from the HBV Pre-S1 region is a fragment of the N-terminus of the pre-S 1 region of the surface antigen of HBV genotype D, or a variant thereof, and the fragment comprises at least the amino acid 2-33 of the N-terminus, preferably comprises at least the amino acid 2-48 of the N-terminus; or
the HBV entry inhibitory polypeptide derived from the HBV Pre-S1 region is a fragment or a variant thereof of the N-terminus of the pre-S1 region of the surface antigen of HBV genotype E, G, and the fragment comprises at least the amino acid 12-43 of the N-terminus, preferably comprises at least the amino acid 12-68 of the N-terminus;
preferably, the variant has 1-30 amino acid deletions, substitutions or insertions compared to the fragment, and the variant retains the biological activity of inhibiting HBV entry or binding to NTCP; preferably, the variant comprises at the N and/or C terminus of the fragment a natural flanking amino acid sequence from any of the HBV subtypes; preferably, the natural flanking amino acid sequence is selected from: the amino acids 2-12 of the N-terminus of the pre-S1 region of the surface antigen of HBV genotypes A, B, F, H, I, and the amino acids 2-11 of the N-terminus of the pre-S1 region of the surface antigen of HBV genotypes E, G; preferably, the variant is a variant that obtained by introducing one or any combination of two or more of the following amino acid substitutions into a derivative peptide derived from the amino acids 13-59 of the Pre-S 1 region of HBV genotype C: N15D, F25L, G35K, N39E, F45L, N46K, N48H or N48Y or N48K, D50A, H51Q or H51N, E54K or E54D, A55S, N56K or N56D, and Q57K, optionally, the N- and/or C-terminus of the variant comprises the natural flanking amino acid sequence of any of the HBV subtype.

4. The use according to claim 3, wherein the amino acid sequence of the fragment is as shown by any one of SEQ ID NO: 2-32, or has at least 30%, 40%, 50%, 60%, 70%, 80%, 85%, 90%, 95%, 96%, 97%, 98% or 99% identity with the amino acid sequence as shown by any one of SEQ ID NO: 2-32.

5. The use according to any one of claims 2-4, wherein the HBV entry inhibitory polypeptide derived from the HBV Pre-S 1 region has N-terminus modification and/or C-terminus modification;
preferably, the N-terminus modification is a hydrophobic group modification; preferably, the hydrophobic group modification is selected from myristic acid, palmitic acid, stearic acid, cholesterol, oleic acid, linoleic acid, polyethylene glycol and arachidonic acid modification;
preferably, the C modification is amidation or isopentyl glycolation modification;
preferably, the HBV entry inhibitory polypeptide derived from the HBV Pre-S1 region is shown by SEQ ID NO: 33-44.

6. The use according to any one of claims 1-5, wherein the nucleoside (acid) medicine is selected from the group consisting of: lamivudine, adefovir dipivoxil, entecavir, tenofovir dipivoxil, telbivudine and tenofovir alafenamide hemifumarate, preferably tenofovir alafenamide hemifumarate.

7. The use according to any one of claims 1-6, wherein the kit comprises 1 dose or more than 2 doses of the medicine, meeting the daily dose of 2.1-10.5 mg, preferably 4.2-8.4 mg, more preferably 4.2 mg of the HBV viral entry inhibitor;
the kit optionally comprises 1 dose or 2 doses of the interferon, and its content meeting the dosage of 1-360 micrograms per week, preferably 30-180 micrograms per week, more preferably 60-135 micrograms per week, more preferably 90 micrograms per week;
preferably, the kit is used for combined administration with the nucleoside (acid) medicine for 1-96 weeks, preferably 12-60 weeks, more preferably 24-48 weeks.

8. A method for limiting or terminating nucleoside (acid) treatment of chronic hepatitis B patients, the method comprising administering chronic hepatitis B patients with HBV virus entry inhibitor and optional immunomodulators in addition to nucleoside (acid) medicines for combined treatment, and after a certain course of combined treatment, nucleoside (acid) treatment, HBV viral entry inhibitor treatment and the immunomodulator treatment are terminated.

9. The method according to claim 8, wherein the nucleoside (acid) medicine is as described in claim 6; the HBV virus entry inhibitor is as described in any one of claims 2-5, and the immunomodulator is as described in claim 2;
preferably, the duration of the combined treatment is 1-96 weeks, preferably 12-60 weeks, more preferably 24-48 weeks;
preferably, before performing the combined treatment, the patient has been administered continuous NAs treatment, preferably has been administered NAs treatment for more than 0 months, more than 3 months, more than 12 months, more than 36 months;
preferably, the daily dose of the HBV viral entry inhibitor is 2.1-10.5 mg, preferably 4.2-8.4 mg, more preferably 4.2 mg;
preferably, the immunomodulator is interferon, and its weekly dose is 1-360 micrograms, preferably 30-180 micrograms, more preferably 60-135 micrograms, more preferably 90 micrograms.

10. The method according to claim 8 or 9, wherein the nucleoside (acid) medicine is tenofovir alafenamide hemifumarate, and the HBV virus entry inhibitor is hepalatide or bulevirtide;
preferably, the treatment period of the hepalatide or bulevirtide is from 1 week to 96 weeks;
preferably, the therapeutic dose of hepalatide or bulevirtide is 2.1 mg to 8.4 mg per day;
preferably, the immunomodulator is PEG interferon, and its dosage is preferably 10-170 ug per week;
preferably, in the method, the nucleoside (acid) medicine is tenofovir alafenamide hemifumarate; hepalatide or bulevirtide is administered for combined treatment, the treatment period is 48 weekly, the dose is 4.2 mg per day; simultaneously, PEG interferon is administered, the dose is 90 ug per week, and the treatment period is 48 weeks.
